# FASCICULE DE BREVET EUROPEEN

(11) **EP 0 920 520 B1**
(45) Date de publication et mention de la délivrance du brevet: **17.01.2007**
(21) Numéro de dépôt: 98933683.9
(22) Date de dépôt: 23.06.1998
(51) Int. Cl.: C12N 15/82, C12N 5/10, A01H 5/00

(54) **SEQUENCE NUCLEOTIDIQUE CODANT POUR UNE ENZYME AYANT UNE ACTIVITE ACETOLACTATE SYNTHASE, CELLULE VEGETALE ET PLANTE LA CONTENANT.**
FÜR EIN ENZYM MIT ACETOLACTAT-SYNTHASE-AKITVITÄT KODIERENDE NUKLEINSÄURESEQUENZ, UND DIESE ENTHALTENDE PFLANZEN.
NUCLEOTIDE SEQUENCE CODING FOR AN ENZYME HAVING A ACETOLACTATE SYNTHASE ACTIVITY, PLANT CELL AND PLANT CONTAINING IT.

(30) Priorité: 25.06.1997 FR 9707918
(43) Date de publication de la demande: 09.06.1999
(73) Titulaire: Hoquet, Michel, 59554 Neuville Saint Remy (FR)
(72) Inventeur: LEVEQUE, Pascal, F-59142 Villers Outreaux (FR)
(74) Mandataire: Warcoin, Jacques
(86) Numéro de dépôt international: PCT/FR1998/001311
(87) Numéro de publication internationale: WO 1999/000509

(56) Documents cités:
- EP-A- 0 257 993
- US-A- 5 633 437
- DEWAELE, E., ET AL.: "Biochemical characterization of chlorosulfuron resistance in Chicorium intybus L. var. Witloof" JOURNAL OF PLANT PHYSIOLOGY, vol. 151, no. 1, juillet 1997, pages 109-114, XP002081330
- DATABASE SCISEARCH INSTITUTE FOR SCIENTIFIC INFORMATION, PHILADELPHIA, PA, US AN 97:528687, JO, K., ET AL.: "Purification and characterization of the recombinant Arabidopsis thaliana acetolactate synthase" XP002056393 & BULLETIN OF THE KOREAN CHEMICAL SOCIETY, vol. 18, no. 6, 20 juin 1997, pages 648-653,
- LAVIGNE, C.,ET AL.: "Monogenic semidominant sulfonylurea resistance in a line of white chicory" PLANT BREEDING, vol. 113, 1994, pages 305-311, XP002081331
- MOURAD, G., ET AL.: "A double mutant allele, csr1-4, of Arabidopsis thaliana encodes an acetolactate synthase with altered kinetics" PLANTA, vol. 196, 1995, pages 64-68, XP002081332
- HERVIEU, F., ET AL.: "A single amino acid change in acetolactate synthase confers resistance to valine in tobacco" MOL. GEN. GENET., vol. 251, 1996, pages 220-224, XP002056387
- TOURNEUR, C., ET AL.: "OVER-EXPRESSION OF ACETOLACTATE SYNTHASE CONFERS RESISTANCE TO VALINE IN TRANSGENIC TOBACCO." PLANT SCIENCE, vol. 88, 1993, pages 159-168, XP002081333
- VERMEULEN, A., ET AL.: "AGROBACTERIUM MEDIATED TRANSFER OF A MUTANT ARABIDOPSIS ACETOLACTATE SYNTHASE GENE CONFERS RESISTANCE TO CHLORSULFURON IN CHICORY CICHORIUM-INTYBUS L." PLANT CELL REPORTS, vol. 11, 1992, pages 243-247, XP002081334
- HATTORI, J., ET AL.: "Multiple resistance to sulfonylureas and imidazolinones conferred by an acethydroxyacid synthase gene with seperate mutations for selective resistance" MOL. GEN. GENET., vol. 232, 1992, pages 167-173, XP002056388

### LISTE DE SEQUENCES

(1) INFORMATIONS GENERALES:
   (i) DEPOSANT:
      (A) NOM: HOQUET-GRAINES
      (B) RUE: 9, RUE DE SAILLY
      (C) VILLE: RAILLENCOURT
      (E) PAYS: FRANCE
      (F) CODE POSTAL: 59554
   (ii) TITRE DE L' INVENTION: SEQUENCE NUCLEOTIDIQUE CODANT POUR UNE ENZYME AYANT UNE ACTIVITE ACETOLACTATE SYNTHASE, CELLULE VEGETALE ET PLANTE LA CONTENANT, ET PROCEDE DE DESHERBAGE DE PLANTES
   (iii) NOMBRE DE SEQUENCES: 1
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 243 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
   AGCCAAAATC GTTCACATCG ACATCGATTC TGCAGAAATC GGTAAGAACA AGCAACCCCA    60
   TGTTTCTGTT TGTGGTGATA TCAAGATCGA TACAAGGGGT CTCAACAAAA TCCTGGAAGA   120
   AAAAGGAGAT GAATTCTCGA TTTCTCATCT GGAGAAGGAT AGCAGACAAA TCAACCCACC   180
   ATACTCAAAC TCGAGATCGA TCTCTATCAT CAGATCATCG TGATGATCGG CGATCATCAT   240
   ACG                                                                 243

## Description

La présente invention se rapporte à des séquences nucléotidiques associées à une tolérance aux herbicides chez des plantes, en particulier du genre Cichorium.

La chicorée (Cichorium intybus) est une plante dont les premiers stades de développement sont lents. En effet, il ne lui faut pas moins de deux mois avant de couvrir le sol. La culture se trouve, alors, en compétition directe avec un nombre important d'adventices, qui, durant cette période, présentent un développement très actif. Les programmes actuels de désherbage chimique ne permettent pas un contrôle correct des mauvaises herbes. En l'absence d'un herbicide efficace et sélectif de la chicorée, il est fréquent de devoir procéder à l'élimination des adventices restants par binage mécanique complété par un sarclage manuel qui engendrent un surcoût très important.

Si l'activité d'un herbicide par rapport à une plante se traduit par sa non toxicité (au moins pour la dose utilisée) envers cette dernière, la spécificité désigne, elle, les catégories de plantes susceptibles d'être détruites par ce produit, en un mot son spectre d'activité.

Un herbicide est donc d'autant plus efficace qu'il affiche une sélectivité restreinte (au mieux la plante cultivée), une spécificité large et une persistance faible, non pénalisante pour les cultures ultérieures.

L'alternative est donc soit de mettre au point un herbicide nouveau, possédant les caractéristiques de spécificité et de sélectivité requises pour une plante donnée, soit de disposer d'une plante modifiée pour être tolérante à un herbicide connu.

La seconde solution présente un avantage certain car elle permet d'éviter de mener l'ensemble des tests de toxicité nécessaires pour la mise sur le marché de ces produits.

Il est donc souhaitable de disposer d'une souche de Cichorium intybus qui ne soit pas sensible à un herbicide largement utilisé.

Le document Lavigne et al. (Plant Breeding 113, 305-311, 1994) décrit le fait que des lignées de Cichorium intybus résistantes au chlorsulfuron peuvent être obtenues, une méthode pour obtenir de telles lignées (voir page 306, "Matérial and Methods), et le fait que la résistance en question implique une (des) mutation(s) dans le gène codant pour l'acétolactate synthase.

C'est pourquoi la présente invention a pour objet une séquence nucléotidique codant pour une enzyme ayant une activité acétolactate synthase (ALS), fonctionnelle dans le genre Cichorium, caractérisée en ce que ladite enzyme présente une affinité réduite pour les composés du type sulfonylurée ou imidazolinone et en ce qu'elle comporte la séquence SEQ ID n°1.

Les imidazolinones sont absorbées par voie foliaire et racinaire et inhibent la synthèse de trois acides aminés : valine, leucine, isoleucine. Elles comprennent notamment l'imazaméthabenz, l'imazapyr et l'imazaquine.

Les sulfonylurées ont un mode d'action similaire et inhibent également la synthèse de Val, Leu et lle. Elles regroupent un grand nombre de molécules, se distinguant par une efficacité importante (quelques grammes de matière active à l'hectare suffisent), ainsi que par une faible toxicité vis-à-vis des autres organismes vivants. Elles présentent un site d'action biochimique maintenant parfaitement connu, l'ALS ou acétolactate synthase.

On peut citer parmi les représentants de ce groupe les composés suivants : bensulfuron-méthyle, chlorsulfuron, metsulfuron, thifensulfuron-méthyle, triasulfuron, tribénuron-méthyle, amidosulfuron, rimsulfuron et nicosulfuron.

La Demanderesse a maintenant trouvé qu'une tolérance à des doses de sulfonylurées, et en particulier de chlorsulfuron, augmentée d'un facteur 1000, peut être obtenue grâce à une mutation de l'enzyme ALS.

Cette enzyme catalyse la première étape commune aux voies de biosynthèse des acides aminés branchés valine, leucine, isoleucine.

Des plantes du genre Cichorium, en particulier Cichorium intybus L var Witloof, portant une telle enzyme, sont tolérantes au chlorsulfuron : cet herbicide est commercialisé par Dupont de Nemours pour le désherbage anti-dicotylèdones des céréales. Des tolérances croisées sont observées vis-à-vis d'autres sulfonylurées et d'imidazolinone.

Les analyses enzymatiques indiquent que l'acquisition de la tolérance est due à l'altération de la sensibilité de l'ALS vis-à-vis de ces inhibiteurs. L'inhibition de 50% de l'activité ALS nécessite, chez le tolérant, une dose de chlorsulfuron 570 fois supérieure à celle produisant le même effet chez le sensible (21 nM). La sensiblité de l'ALS issue de la lignée tolérante vis-à-vis de l'imazaméthabenz est 125 fois plus faible. Cette altération de l'ALS se transmet de génération en génération, comme un caractère mendelien codé par un gène nucléaire unique. L'analyse du génome des chicorées tolérantes confirme ce caractère monogénique. Par ailleurs, aucune surproduction de cette cible n'a été constatée.

L'activité de l'ALS est connue pour être régulée négativement par les produits finaux, valine et leucine, de la voie de biosynthèse qu'elle initie. L'ALS extraite des chicorées tolérantes présente une diminution de la sensibilité (10 fois pour la leucine et 3 fois pour la valine) à cc rétrocontrôle.

C'est pourquoi, selon un mode de réalisation préféré, l'enzyme ALS présente une affinité réduite pour les acides aminés suivants : leucine, valine.

De manière inattendue, l'activité spécifique de l'ALS tolérante aux sulfonylurées est diminuée par rapport à celle de plantes sensibles.

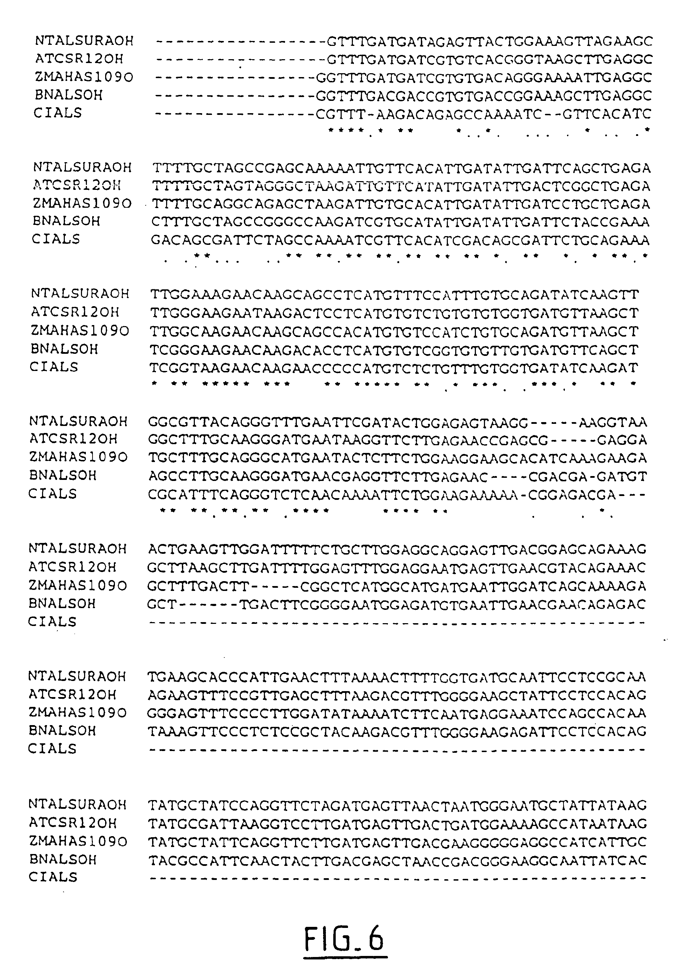

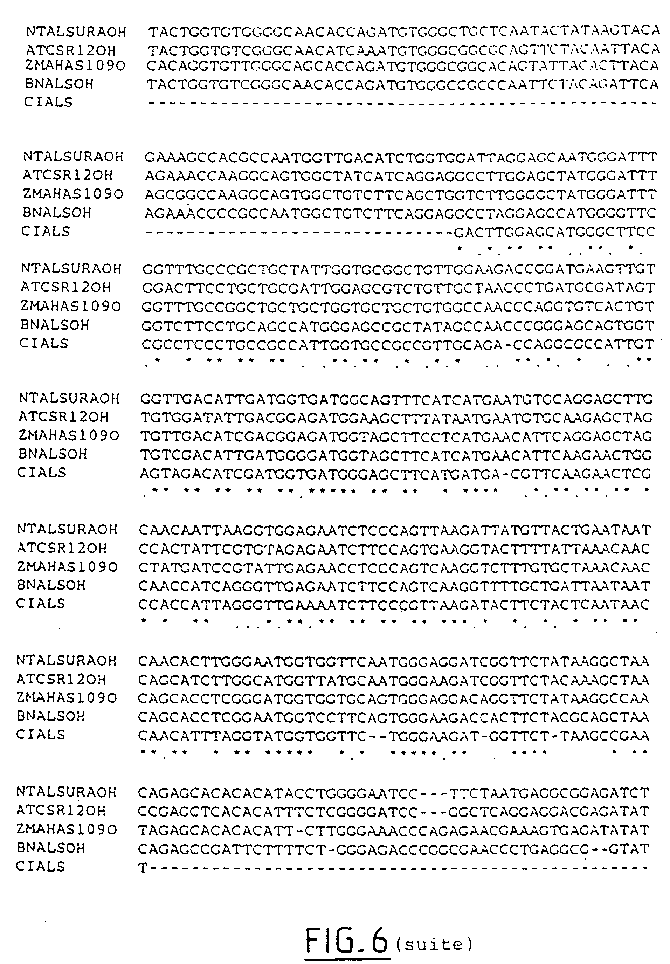

Des gènes codant pour des ALS tolérantes aux sulfonylurées ont été mis en évidence auparavant dans d'autres espèces, comme Nicotiana tabacum, Arabidopsis thaliana. Zea mays ou Brassica napus. Cependant, l'alignement des séquences nucléotidiques permet de distinguer le gène codant pour l'ALS chez différentes espèces et de mettre en évidence le polymorphisme de séquences. L'alignement de 25% de ce gène est représenté sur la figure 6. De plus, la comparaison de séquence entre des fragments de gènes provenant de Cichorium intybus permet de distinguer les gènes codant pour l'ALS issus respectivement de lignée sensible et tolérante.

De manière encore préférée, la séquence nucléotidique selon l'invention code pour une enzyme ALS ayant une activité spécifique diminuée par rapport à l'enzyme naturelle.

Selon un autre aspect, l'invention a pour objet une enzyme acétolactate synthase susceptible d'être codée par une séquence nucléotidique selon l'invention.

Une cellule végétale caractérisée en ce qu'elle contient une séquence nucléotidique telle que définie précédemment, avec les éléments nécessaires à l'expression de ladite séquence et/ou en ce qu'elle contient une enzyme selon l'invention, constitue un autre des objets de l'invention.

De préférence, une telle cellule contient une concentration en leucine et valine augmentée par rapport à la concentration de ces acides aminés dans une cellule sauvage ; ceci résulte de la diminution de sensibilité de l'enzyme selon l'invention au rétrocontrôle par ces acides aminés. Cette cellule végétale, de préférence, est une cellule de Cichorium intybus.

Une souche de chicorée Witloof (Chichorium intybus L. var Witloof) tolérance aux sulfonylurées et/ou aux imidazolinones a pu être obtenue.

En l'absence de tout agent mutagène, des cals tolérants, initiés à partir de suspensions cellulaires, ont été sélectionnés sur milieu de culture renfermant 28 nM de chlorsulfuron (dose léthale pour les chicorées non-sélectionnées). Ces derniers ont permis la régénération de plantes fertiles capables de supporter des doses d'herbicides 1500 à 2000 fois supérieures pour les individus homozygotes, et 300 fois supérieures pour les individus hétérozygotes. En l'absence d'herbicide, aucun coût consécutif à l'acquisition de la tolérance n'est mesurable, tant sur le développement végétatif, que sur les potentialités reproductrices de la lignée tolérante.

Ainsi, un autre objet de l'invention concerne un procédé de sélection de plantes modifiées pour être tolérantes à un herbicide et comportant une séquence nucléotidique selon l'invention utilisant un herbicide inhibant l'enzyme ALS.

L'invention a donc également pour objet une plante obtenue par un procédé essentiellement microbiologique, qui contient une séquence nucléotidique telle que définie ci-dessus, ladite plante étant tolérante aux herbicides du groupe des sulfonylurées et des imidazolinones.

Selon un autre de ses aspects, l'invention concerne une méthode de détection d'une plante tolérante aux herbicides du groupe des sulfonylurées et des imidazolinones, caractérisée en ce qu'elle comprend une étape au cours de laquelle on met en évidence dans du matériel végétal issu d'une plante que l'on veut tester, la présence d'une séquence nucléotidique selon l'invention.

Selon un-mode de mise en oeuvre, cette détection peut être effectuée à l'aide d'amorces, correspondant à des fragments situés respectivement aux extrémités N et C terminales de la séquence en cause.

Selon un autre mode de mise en oeuvre, on met en contact du matériel végétal issu d'une, plante que l'on veut tester, avec une sonde selon l'invention, et que l'on vérifie s'il y a hybridation.

Enfin, un autre des objets de l'invention est un procédé de désherbage de plantes à l'aide d'un herbicide inhibant l'enzyme ALS, dans lequel une plante modifiée pour être tolérante à l'herbicide comporte une séquence nucléotidique selon l'invention.

Les exemples qui suivent sont destinés à illustrer l'invention.

Dans ces exemples on se référera aux figures suivantes :

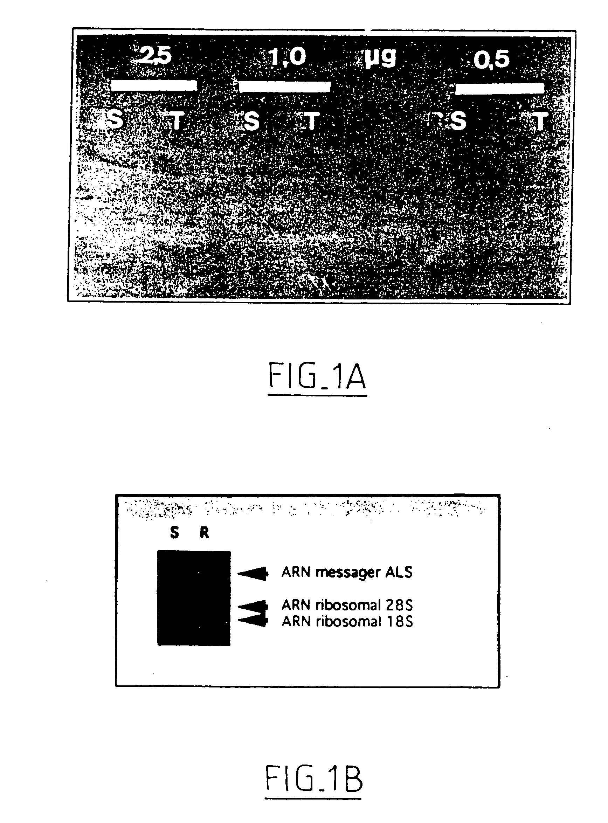

- **Figure 1** :: A : Comparaison des teneurs en protéine ALS chez les souches sauvages (S) et tolérante (T). La quantité de protéines totales déposées dans chaque condition est indiquée en µg.
B : Comparaison de l'activité transcriptionnelle chez les souches sauvages (S) et tolérante (R).
Analyse en Nothernblot de l'expression de l'ARN messager correspondant à l'ALS de lignée de chicorées sensibles et tolérantes. L'ARN total a été extrait et 10 µg de chaque échantillon ont été soumis à électrophorèse, transférés sur une membrane nylon et révélés par un fragment d'ALS de chicorée marqué au 32P.

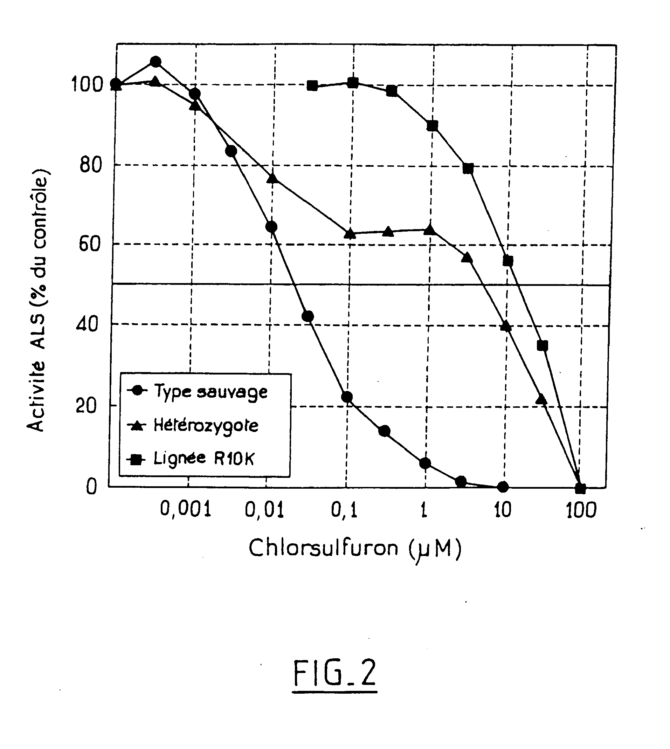

- **Figure 2 :**: Inhibition de l'activité enzymatique ALS extraite de plantes sensibles, tolérantes et hétérozygotes (femelle sensible x mâle tolérant), par le chlorsulfuron. Les valeurs sont exprimées comme en pourcentage des contrôles non traités, et représentent la moyenne des résultats obtenus dans trois expériences indépendantes.

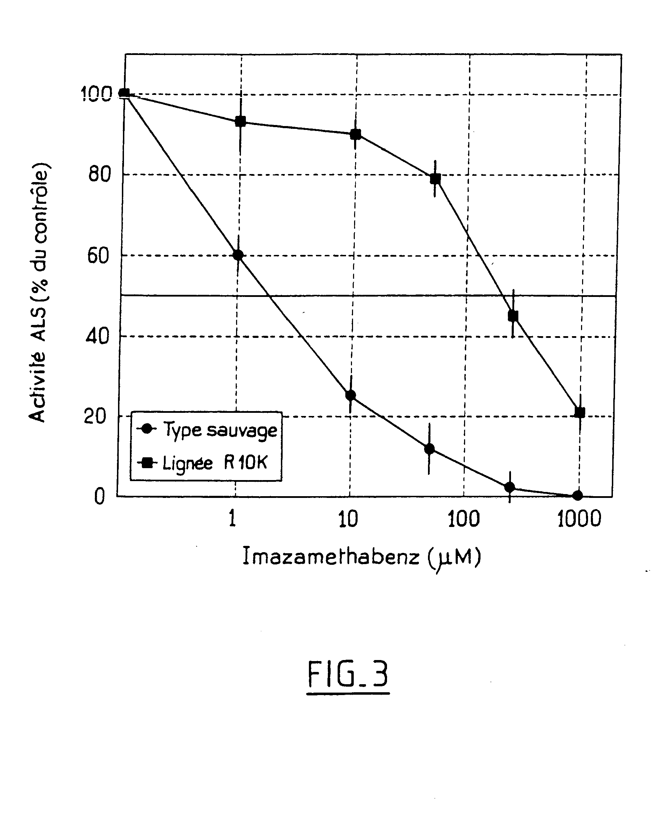

- **Figure 3 :**: Effet de concentration croissante d'imadazolinone-imazaméthabenz sur l'activité ALS extraite de chicorées de type sauvage et tolérantes au chlorsulfuron. Les valeurs, exprimées en pourcentage des contrôles non traités, sur la moyenne des résultats obtenus avec deux préparations enzymatiques différentes.

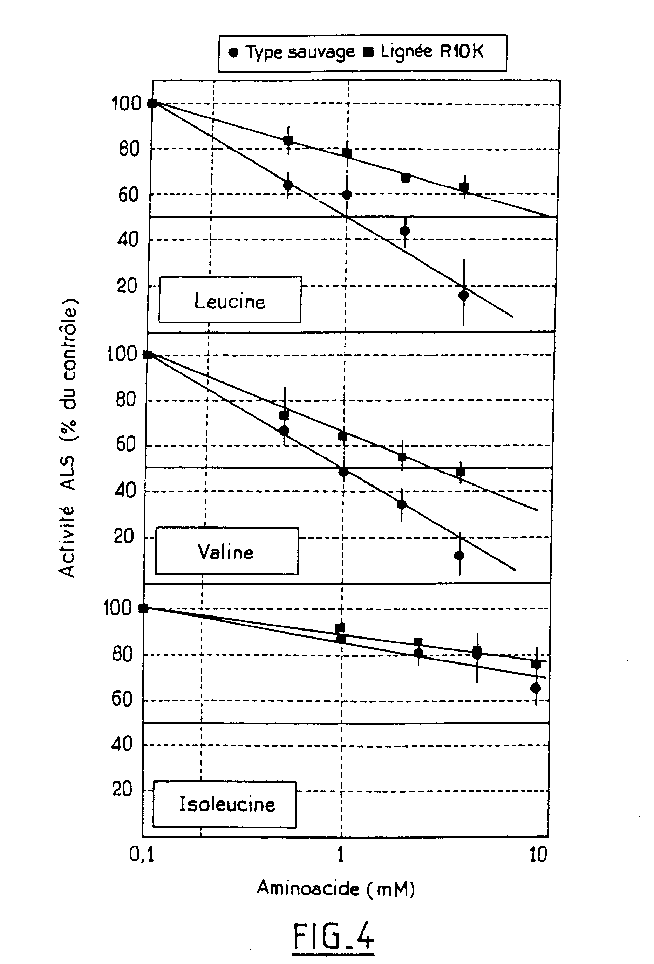

- **Figure 4** :: Conséquence de la mutation sur le rétrocontrôle de l'ALS par les acides aminés branchés. La sensibilité d'ALS extraite de chicorées tolérantes au chlorsulfuron et de type sauvage à l'inhibition par rétrocontrôle par chacun des trois amino acides branchés a été évaluée en mesurant l'activité enzymatique en présence de concentration croissante d'isoleucine, de leucine et de valine. Les résultats, exprimés comme pourcentage de contrôles non traités, sont les moyennes de données obtenues dans trois expériences indépendantes.

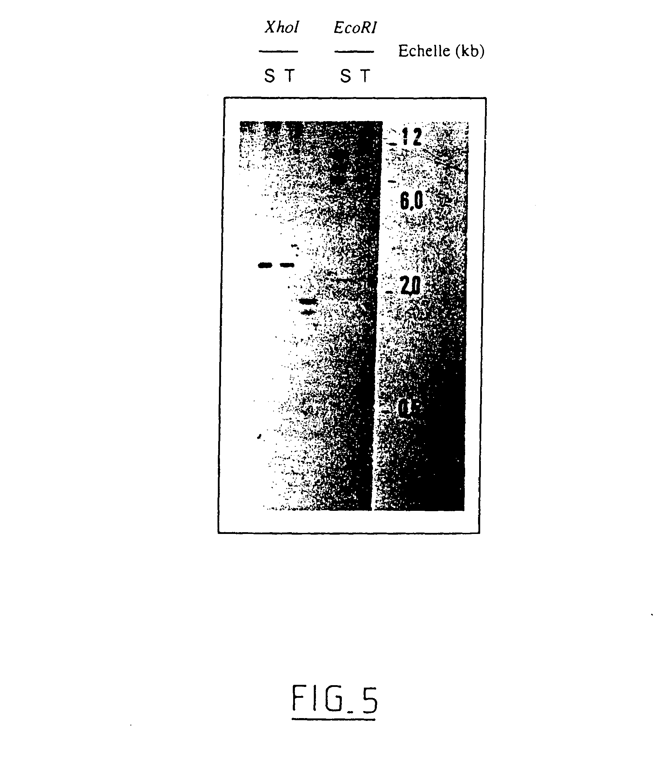

- **Figure 5 :**: Analyse du génome des chicorées tolérantes digéré par des enzymes de restrictition EcoRI et Xho1 et hybridé à l'aide d'un fragment du gène codant pour l'ALS de chicorée. Ce fragment renferme un site EcoRI mais pas de site Xho1.
- **Figure 6 :**: Polymorphisme de séquences nucléotidiques des gènes codant pour les ALS de différentes espèces (l'alignement ne porte que sur 25% du gène).
Les alignements de séquences représentés sont destinés à mettre en évidence le polymorphisme de séquence observé pour le gène codant pour l'ALS chez différentes espèces. Le caractère * montre les positions parfaitement bien conservées, alors que le caractère montre les positions bien conservées.
Liste des séquences utilisées pour l'alignement :
[1] NTALSURAOH : Gène codant pour l'acétolactate synthase de Nicotiana tabacum.
[2] ATCSR12OH : gène codant pour l'acétolactate synthase d'arabidopsis thaliana.
[3] ZMAHAS1090 : gène codant pour l'acétolactate sythase de Zea mays.
[4] BNALSOH : gène codant pour l'acétolactate synthase de Brassica napus.
[5] CIALS : gène codant pour l'acétolactate synthase de Chicorium intybus.

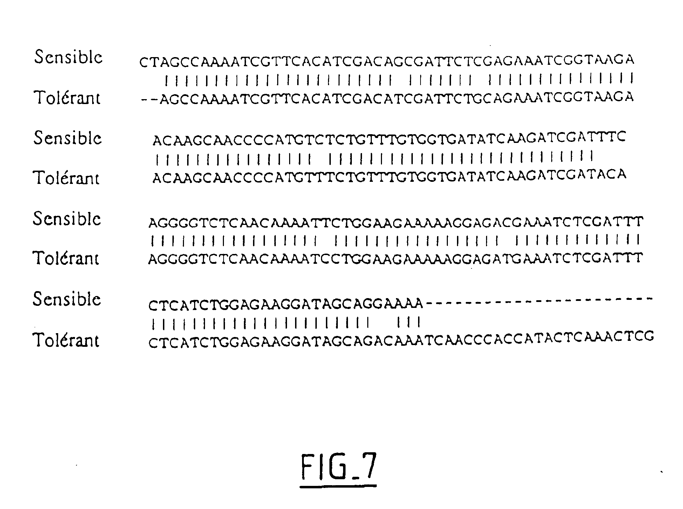

- **Figure 7 :**: Polymorphisme intraspécifique : comparaison de séquences entre fragments de gènes codant pour l'ALS, issus de lignées sensibles et tolérantes.

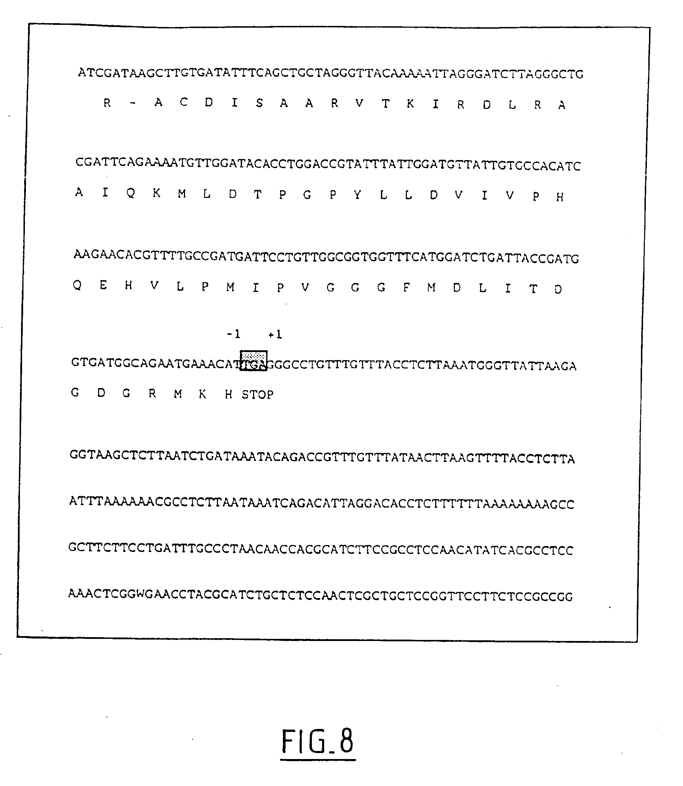

- **Figure 8 :**: Séquence terminale du gène codant pour l'ALS de chicorée sensible et sa traduction en acides aminés.

### Exemple 1 : Obtention d'une lignée résistante au chlorsulfuron

De jeunes plantules issus de germination aseptique d'akènes ont été utilisés pour obtenir cette lignée, des suspensions cellulaires sont mises en culture puis jusqu'à obtention d'explants calogènes. Les cals et les cellules cultivés en milieu liquide agité sont prélevés après 15 jours de culture et étalés sur un milieu gélosé contenant l'herbicide, ainsi que sur un milieu non sélectif.

Après 30 à 40 jours de culture, les amas cellulaires non nécrosés en présence des herbicides sont repiqués sur un milieu de sélection de même composition. Cette opération est répétée jusqu'à ce qu'il soit possible de selectionner des cellules capables de se multiplier normalement en présence d'herbicide. Ces cultures sont alors déposées sur un milieu sélectif avec des cellules témoins n'ayant jamais subi de pression de sélection de façon à comparer le comportement de souches sensibles ou supportant l'herbicide.

On produit ensuite des vitroplants en maintenant les cals sur les milieux d'induction jusqu'à ce qu'ils forment des nodules méristématiques puis en repiquant sur un milieu de développement des bourgeons. Les jeunes vitroplants une fois enracinés sont prélevés et repiqués en mini serres. On isole ainsi une souche R10K qui se développe normalement en présence de chlorsulfuron et de sulfométuron.

### Exemple 2 : caractérisation de la résistance au chlorsulfuron

### 1. Matériels et Méthodes

### - Matériel végétal

Des graines obtenues à partir de chicorée du type sauvage, homozygote résistant au chlorsurfuron (R1OK) et hétérozygote (obtenues par pollinisation de plantes de type sauvage avec du pollen R10K) ont été stérilisées en surface pendant 15 min dans une solution aqueuse de chlorure mercurique à 0,1%, rincées trois fois à l'eau distillée stérile et mises à germer sur des sels de Heller agarisés (Heller, 1953) contenant 20 g/l de saccharose. Les plantules sont cultivées à 22 ± 1° C, sous un rythme de 16 heures de jour (250 µE m⁻² s⁻¹ densité du fluide photo photosynthétique) et 8 heures de nuit. Après un mois, les plantes sont récoltées et utilisées pour l'extraction d'ARN et l'analyse enzymatique.

### - Extraction d'acétolactate synthase et dosage

L'activité ALS a été extraite et dosée comme décrit par Forlani et al (Plant Growth Regul. 14, 203-209, 1994). En bref, 5 g de feuilles sont réduites en poudre dans de l'azote liquide et remises en suspension dans 5 ml.g⁻¹ du tampon phosphate 50 mM (pH 7,5) contenant 0,5 mM dithiothréitol, 1 mM MgCl₂, 0,1 mM pyrophosphate de thiamine (TPP), 0,01 mM de dinucléotide flavine adénine (FAD), 20% de glycérol et 2% (p/v) de polyvinyl polypyrrolidone. Après centrifugation pendant 20 minutes à 18 000 g, le surnageant est additionné de sulfate d'ammonium solide (70% de la saturation) et centrifugé. Les culots sont dissous dans du tampon d'extraction à demi force et désalés par passage à travers une colonne de Sephadex G25 (Pharmacia). L'activité enzymatique est mesurée comme la quantité d'acétolactate produite. Les aliquots correspondant à environ 5 pkat d'activité ALS sont mis à incuber jusqu'à 90 min à 35° C en présence de tampon phosphate potassium 20 mM (pH 7,5) pyruvate de sodium 40 mM, MgCl₂ 1 mM, TPP 0,1 mM et FAD 0,01 mM. Après décarboxylation, catalysée à l'acide, de l'acétolactate en acétoïne, cette dernière est déterminée par colorimétrie. Des témoins sont effectués pour quantifier le bruit de fond. Les moyennes des activités spécifiques de l'ALS sont calculées sur au moins cinq déterminations indépendantes sur des semis ayant une croissance uniforme, récoltés au même stade de développement. L'inhibition enzymatique a été estimée par addition de concentration croissante de chlorsulfuron, d'imazaméthabenz, d'isoleucine, leucine et valine au milieu de réaction ; trois mesures ont été effectuées pour chaque dosage. Chaque expérience a été répétée deux fois sur différentes préparations enzymatiques : les moyennes des deux répétitions sont présentées, exprimées par un pourcentage des contrôles non traités.

### - Analyse par Northern blot

L'ARN total a été extrait et des aliquots de 10 µg ont été dénaturés et soumis à électrophorèse sur des gels d'agarose 1,5%, contenant 3% de formaldéhyde. Les gels sont buvardés sur des membranes Hybon-N avec 20 X SSC. La sonde chicorée utilisée pour la détection du transcrit d'ALS a été générée par PCR, avec des amorces homologues à la séquence du gène sur A du tabac, comprenant les positions 1274 à 1295 et 1883 à 1902 (Mazur et al, Plant Physiol. 85, 1110-1117, 1987). Le fragment de 630 pb obtenu a été subcloné en PCR^{™} II (Invitrogene) utilisant des procédures de clonage classiques et séquencé par la procédure de terminaison didéoxy (Sanger et al, 1977, Proc Natl. Acad. Sci. USA 74, 5463-5467, 1977). La sonde a été marquée au 32P en utilisant le kit T7-Quick-Prime (promega) selon les indications du fabricant. L'hybridation a été effectuée à 60° C. Les autoradiogrammes ont été analysés sur un densitomètre Microtek Color/Gary^{™} (Biorad) et les données traitées avec le programme Cricket Graph^{™}.

### 2. Résultats et discussion

### Tolérance à l'herbicide

Les premiers élément pris en considération sont la quantité d'ARN messager-ALS et le niveau d'activité spécifique ALS. Les expériences de Northern blot effectuées avec aussi bien des plantes sensibles que résistantes n'ont pas montré de différence substantielle entre les deux lignées (figure 1). Au contraire, l'analyse enzymatique a révélé une différence significative quant à l'activité spécifique ALS, qui est de 12,2 ± 1,1 pkat.mg⁻¹ de protéine totale dans le type sauvage et 7,9 ± 0,8 pkat.mg⁻¹ de protéine dans les plantes résistantes. Ces résultats excluent la possibilité que la résistance soit dérivée partiellement ou complètement d'une augmentation du taux d'ALS. On peut noter que malgré une diminution de 35% de l'activité enzymatique spécifique, les plantes résistantes ne montrent pas de réduction ou d'altération de caractéristiques végétatives ou reproductives. Etant donné que le niveau d'ARNm n'est pas modifié, une explication possible pour cette réduction pourrait être la survenue d'une mutation affectant la stabilité de l'enzyme.

### Comparaison de la sensibilité in vitro au chlorsulfuron entre des AIS provenant de chicorées résistant aux herbicides et sensibles

L'activité ALS de plantes résistantes sensibles a été dosée en présence de concentrations croissantes de chlorsulfuron. Les résultats ont montré que l'ALS de plantes résistantes n'est pas inhibée jusqu'à des concentrations en herbicide de l'ordre du micromolaire, alors que celle provenant de plantes sauvages était déjà affectée par des taux nanomolaires (figure 2). La concentration requise pour inhiber 50% de l'activité ALS de plantes résistantes est de 12 µM, alors que la DI₅₀ pour l'enzyme de plantes du type sauvage est de 0,021 µM, correspondant à une différence de 570 fois. Des comportements différents de façon si marquée suggèrent fortement une sensibilité diminuée de l'ALS à l'effet inhibiteur du chlorsulfuron comme déterminant de la tolérance sélectionnée.

L'ALS a également été extraite de chicorée hétérozygote obtenue par pollinisation de plantes de type sensible avec du pollen R10K, et l'activité enzymatique a été mesurée en présence de l'herbicide. Les résultats montrent un profil biphasique (figure 2), indiquant la présence concurrente d'une forme d'ALS résistante avec une forme sensible.

### L'enzyme résistant au chlorsulfuron montre une tolérance croisée envers l'herbicide imidazolinone : imazaméthabenz

L'activité ALS respectivement des types sauvage et résistant a été mesurée en présence de concentrations croissantes d'imazaméthabenz. Comme on le voit sur la figure 3, l'activité ALS de plantes résistantes n'a pas été affectée à des concentrations allant jusqu'à 10 µM, la DI₅₀ étant d'environ 200 µM, alors que pour l'ALS provenant de plantes de type sauvage, l'activité a été inhibiée à 50% à 1,8 µM. Sélectionnée initialement pour sa résistance au chlorsulfuron, la lignée R10K apparait ainsi dotée d'une activité ALS qui est aussi 125 fois plus résistante à un herbicide appartenant à une autre classe d'inhibiteurs d'ALS. Dans le mutant de chicorée, la différence dans le niveau de résistance au chlorsulfuron et à l'imazaméthabenz est apparemment en accord avec l'existence d'un site unique de mutation avec des domaines de fixation spécifiques se recouvrant.

### Sensibilité au rétrocontrôle de l'ALS par la leucine et la valine.

Les acides aminés à chaîne ramifiée modulent leur propre synthèse en inhibant de façon coopérative la première enzyme de leur voie de biosynthèse. L'activité ALS a ainsi été mesurée dans des extraits provenant de plantes de type sauvage et R10K en présence de concentrations croissantes de valine, leucine, ou isoleucine. Comme on s'y attendait, la valine et la leucine diminuent de façon marquée l'activité de l'ALS provenant du type sauvage avec des taux d'inhibition similaires (DI₅₀ environ 1 mM) alors que l'isoleucine, qui contrôle le flux de carbone à travers sa voie au niveau de la thréonine désaminase est substantiellement inefficace (DI₅₀ supérieure à 100 mM ; figure 4). Quand l'activité résistante au chlorsulfuron extraite de chicorée R10K a été examinée, elle a été trouvée significativement moins sensible à l'effet inhibiteur de la valine (DI₅₀ = 3 mM), ou encore plus de la leucine (DI₅₀ aux environs de 10 mM ; figure 4). Il est donc probable que la forme résistante de l'ALS de la lignée R10K résulte d'un ou plusieurs événements mutationnels qui concernent à la fois le site de fixation aux herbicides et le domaine impliqué dans le rétrocontrôle par la valine et la leucine.

Cette diminution de sensibilité au rétrocontrôle (10 fois pour la leucine, et 3 fois pour la valine) se traduit par une augmentation des teneurs en valine (+140%) et leucine (+80%) libres dans ces plantes, comme cela apparaît sur le tableau suivant :

**Teneur exprimée en nmol.g⁻¹ de matière fraiche :**

| Acide aminé | Sensible | Tolérant |
|---|---|---|
| Valine | 10,1 ± 1,4 | 24,3 ± 1,8 |
| Leucine | 9,0 ± 1,9 | 16,0 ± 1,6 |
| Isoleucine | 6,9 ± 1,1 | 8,2 ± 1,2 |
| Total (20 aa) | 1502 | 1600 |

## Revendications

1. Séquence nucléotidique codant pour une enzyme ayant une activité acétolactate synthase (ALS), fonctionnelle dans le genre Cichorium, **caractérisée en ce que** ladite enzyme présente une affinité réduite pour les composés du type sulfonylurée ou imidazolinone et **en ce qu'**elle comporte la séquence SEQ ID N°1.

2. Séquence nucléotidique selon la revendication 1, **caractérisée en ce que** l'enzyme ALS présente une affinité réduite pour les acides aminés suivants : leucine, valine.

3. Séquence nucléotidique selon l'une des revendications 1 ou 2, **caractérisée en ce qu'**elle code pour une enzyme ALS ayant une activité spécifique diminuée par rapport à l'enzyme naturelle.

4. Enzyme acétolactate synthase susceptible d'être codée par une séquence nucléotidique selon l'une des revendications 1 à 3.

5. Cellule végétale **caractérisée en ce qu'**elle contient une séquence nucléotidique selon l'une des revendications 1 à 3, avec les éléments nécessaires à l'expression de ladite séquence et/ou **en ce qu'**elle contient une enzyme selon la revendication 3.

6. Cellule végétale selon la revendication 5, **caractérisée en ce qu'**elle contient une concentration en leucine et en valine augmentée par rapport à la concentration de ces acides aminés dans une cellule sauvage.

7. Cellule végétale selon l'une des revendications 5 à 6, **caractérisée en ce qu'**elle est une cellule de Cichorium intybus.

8. Plante obtenue par un procédé essentiellement microbiologique, **caractérisée en ce qu'**elle contient une séquence nucléotidique SEQ ID N°1, et **en ce qu'**elle est tolérante aux herbicides du groupe des sulfonylurées et des imidazolinones.

9. Méthode de détection d'une plante tolérante aux herbicides du groupe des sulfonylurées et des imidazolinones, **caractérisée en ce qu'**elle comprend une étape au cours de laquelle on met en évidence dans du matériel végétal issu d'une plante que l'on veut tester, la présence d'une séquence nucléotidique selon l'une des revendications 1 à 3.

10. Procédé de sélection de plantes modifiées pour être tolérantes à un herbicide et comportant une séquence nucléotidique selon l'une quelconque des revendications 1 à 3 utilisant un herbicide inhibant l'enzyme ALS.

## Claims

1. Nucleotide sequence encoding an enzyme having an acetolactate synthase (ALS) activity, that is functional in the *Cichorium* genus, **characterized in that** said enzyme exhibits a reduced affinity for compounds of the sulphonylurea or imidazolinone type, and **in that** it comprises the sequence SEQ ID No. 1.

2. Nucleotide sequence according to Claim 1, **characterized in that** the ALS enzyme exhibits a reduced affinity for the following amino acids: leucine, valine.

3. Nucleotide sequence according to either of Claims 1 and 2, **characterized in that** it encodes an ALS enzyme having a decreased specific activity compared with the natural enzyme.

4. Acetolactate synthase enzyme that may be encoded by a nucleotide sequence according to one of Claims 1 to 3.

5. Plant cell, **characterized in that** it contains a nucleotide sequence according to one of Claims 1 to 3, with the elements required for the expression of said sequence, and/or **in that** it contains an enzyme according to Claim 3.

6. Plant cell according to Claim 5, **characterized in that** it contains an increased concentration of leucine and of valine compared with the concentration of these amino acids in a wild-type cell.

7. Plant cell according to either of Claims 5 and 6, **characterized in that** it is a *Cichorium intybus* cell.

8. Plant obtained by means of an essentially microbiological method, **characterized in that** it contains a nucleotide sequence SEQ ID No. 1, and **in that** it is tolerant to herbicides of the sulphonylurea and imidazolinone group.

9. Method of detecting a plant tolerant to herbicides of the sulphonylurea and imidazolinone group, **characterized in that** it comprises a step during which the presence of a nucleotide sequence according to one of Claims 1 to 3 is demonstrated in plant material derived from a plant that it is desired to test.

10. Method of selecting plants modified so as to be tolerant to a herbicide and containing a nucleotide sequence according to any one of Claims 1 to 3, using an ALS enzyme-inhibiting herbicide.

## Patentansprüche

1. Nukleotidsequenz, die ein Enzym mit einer Acetolactatsynthase (ALS)-Aktivität, welche in der Gattung Cichorium funktionsfähig ist, kodiert, **dadurch gekennzeichnet, dass** das Enzym eine verringerte Affinität für die Verbindungen vom Sulfonylharnstoff- oder Imidazolinon-Typ aufweist und dass sie die Sequenz SEQ ID Nr. 1 umfasst.

2. Nukleotidsequenz nach Anspruch 1, **dadurch gekennzeichnet, dass** das ALS-Enzym eine verringerte Affinität für die folgenden Aminosäuren aufweist: Leucin, Valin.

3. Nukleotidsequenz nach einem der Ansprüche 1 oder 2, **dadurch gekennzeichnet, dass** sie ein ALS-Enzym mit einer bezogen auf das natürliche Enzym verringerten spezifischen Aktivität kodiert.

4. Acetolactatsynthase-Enzym, welches durch eine Nukleotidsequenz nach einem der Ansprüche 1 bis 3 kodiert werden kann.

5. Pflanzliche Zelle, **dadurch gekennzeichnet, dass** sie eine Nukleotidsequenz nach einem der Ansprüche 1 bis 3 mit den für die Expression der Sequenz erforderlichen Elementen enthält und/oder dass sie ein Enzym nach Anspruch 3 enthält.

6. Pflanzliche Zelle nach Anspruch 5, **dadurch gekennzeichnet, dass** sie eine Konzentration an Leucin und an Valin enthält, die bezogen auf die Konzentration dieser Aminosäuren in einer wilden Zelle erhöht ist.

7. Pflanzliche Zelle nach einem der Ansprüche 5 bis 6, **dadurch gekennzeichnet, dass** sie eine Cichorium intybus-Zelle ist.

8. Pflanze, die durch ein im Wesentlichen mikrobiologisches Verfahren erhalten wird, **dadurch gekennzeichnet, dass** sie eine Nukleotidsequenz SEQ ID Nr. 1 enthält und dass sie gegenüber den Herbiziden aus der Gruppe der Sulfonylharnstoffe und der Imidazolinone tolerant ist.

9. Verfahren zum Nachweis einer gegenüber den Herbiziden aus der Gruppe der Sulfonylharnstoffe und der Imidazolinone toleranten Pflanze, **dadurch gekennzeichnet, dass** es einen Schritt umfasst, während welchem man in pflanzlichem Material, welches von einer Pflanze, die man testen will, stammt, das Vorhandensein einer Nukleotidsequenz nach einem der Ansprüche 1 bis 3 nachweist.

10. Verfahren zur Selektion von Pflanzen, die modifiziert sind, um gegenüber einem Herbizid tolerant zu sein, und eine Nukleotidsequenz nach einem der Ansprüche 1 bis 3 umfassen, unter Verwendung eines Herbizids, welches das Enzym ALS inhibiert.
